# EUROPÄISCHE PATENTSCHRIFT

(11) **EP 0 365 907 B1**
(45) Veröffentlichungstag und Bekanntmachung des Hinweises auf die Patenterteilung: **22.12.1993**
(21) Anmeldenummer: 89118735.3
(22) Anmeldetag: 09.10.1989
(51) Int. Cl.: C07D 233/64

(54) **Verfahren zur Herstellung von p-Nitrophenyl-imidazolen**
Process for the preparation of p-nitrophenyl imidazoles
Procédé de préparation de p-nitrophényl-imidazoles

(30) Priorität: 15.10.1988 DE 3835195
(43) Veröffentlichungstag der Anmeldung: 02.05.1990
(73) Patentinhaber: BASF Aktiengesellschaft, 67063 Ludwigshafen (DE)
(72) Erfinder: Koehler, Hermann, Dr., D-6719 Bobenheim (DE); Ebel, Klaus, Dr., D-6704 Mutterstadt (DE); Karn, Helmut, D-6700 Ludwigshafen (DE); Taubitz, Christof, Dr., D-6706 Wachenheim (DE)

(56) Entgegenhaltungen:
- JOURNAL OF THE CHEMICAL SOCIETY, 1. Januar 1963, Letchworth, GB, Seiten 1363-1370; T. VAN ES et al.: "Substitution of 4,5-diphenyl-oxazoles and -imidazoles, and some related compounds"

## Beschreibung

Die vorliegende Erfindung betrifft ein Verfahren zur Herstellung von p-Nitrophenyl-imidazolen.

Aus J. Chem. Soc. 1363 bis 1370 (1963) ist die Herstellung von 4,5-Bis-(p-nitrophenyl)-imidazolverbindungen aus 4,5-Diphenyl-imidazolverbindungen mit Nitriersäure bekannt. Die Ausbeuten an 4,5-Bis-(p-nitrophenyl)-imidazolen lassen jedoch zu wünschen übrig.

Aus Heterocycles 27, Seiten 371 bis 376 (1988) ist bekannt, daß die Nitrierung von 4(5)-Phenylimidazol überwiegend zu dem übernitrierten 4-p-Nitrophenyl-5-nitro-imidazol führt, auch dann, wenn man Salpetersäure in stöchiometrischen Mengen einsetzt. 4(5)-p-Nitrophenyl-imidazol entsteht nur als Nebenprodukt.

Der vorliegenden Erfindung lag daher die Aufgabe zugrunde, einen besseren Zugang zu den p-Nitrophenyl-imidazolen zu finden, und den Nachteilen der bekannten Verfahren abzuhelfen.

Demgemäß wurde ein Verfahren Zur Herstellung von p-Nitrophenyl-imidazolen der allgemeinen Formel I 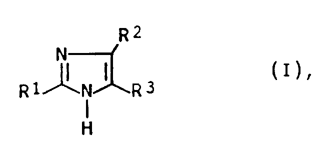
in der R¹ bis R³ unabhängig voneinander Wasserstoff, Alkyl, Cycloalkyl oder p-Nitrophenyl bedeuten, mit der Maßgabe, daß mindestens einer der Reste R¹ bis R³ für p-Nitrophenyl steht, durch Umsetzung von Phenylimidazolen der allgemeinen Formel II 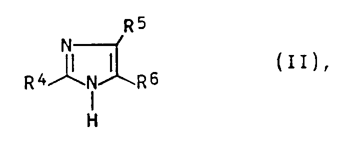
in der R⁴ bis R⁶ unabhängig voneinander Wasserstoff, Alkyl, Cycloalkyl, Phenyl oder p-Nitrophenyl bedeuten, mit der Maßgabe, daß mindestens einer der Reste R⁴ bis R⁶ für Phenyl steht, mit Gemischen von Schwefelsäure und Salpetersäure gefunden, welches dadurch gekennzeichnet ist, daß man die Reaktion in Gegenwart von 0,02 bis 3 Mol Harnstoff pro Mol Phenylimidazol durchführt.

Phenylimidazolverbindungen sind bekannt oder können nach literaturüblichen Verfahren, wie z.B. in J. Chem. Soc. 49, 462 bis 472 (1886) oder J. Org. Chem. 2, 319 bis 326 (1938) beschrieben, hergestellt werden.

Bevorzugt sind Phenylimidazole der allgemeinen Formel II 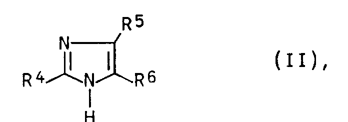
in der R⁴ bis R⁶ unabhängig voneinander Wasserstoff, Alkyl, Cycloalkyl, Phenyl oder p-Nitrophenyl bedeuten, mit der Maßgabe, daß mindestens einer der Reste R⁴ bis R⁶ für Phenyl steht, die übrigen können gleich oder verschieden sein und unabhängig voneinander Wasserstoff, unverzweigtes oder verzweigtes Alkyl mit 1 bis 18 C-Atomen, Cycloalkyl mit 5 bis 8 C-Atomen, Phenyl oder p-Nitrophenyl bedeuten.

P-Nitrophenyl-imidazole der allgemeinen Formel I 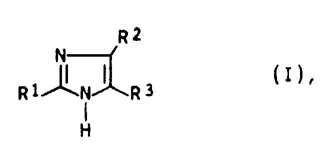
in der R¹ bis R³ unabhängig voneinander Wasserstoff, Alkyl, Cycloalkyl oder p-Nitrophenyl bedeuten, mit der Maßgabe, daß mindestens einer der Reste R¹ bis R³ für p-Nitrophenyl steht, sind bevorzugt; die übrigen Reste können gleich oder verschieden sein und unabhängig voneinander Wasserstoff, unverzweigtes oder verzweigtes Alkyl mit 1 bis 18 C-Atomen, Cycloalkyl mit 5 bis 8 C-Atomen oder p-Nitrophenyl bedeuten.

Phenylimidazole der Formel II sind z.B. 2-Phenylimidazol, 4(5)-Phenylimidazol, 2-Phenyl-4(5)-methylimidazol, 2-Methyl-4(5)-phenylimidazol, 2-Ethyl-4(5)-phenylimidazol, 4,5-Diphenylimidazol, 4(5)-Methyl-2,5(4)-diphenylimidazol, 2-Methyl-4,5-diphenylimidazol, 2-Ethyl-4,5-diphenylimidazol, 2-Propyl-4,5-diphenylimidazol, 2-Isopropyl-4,5-diphenylimidazol, 2-Butyl-4,5-diphenylimidazol, 2-Cyclopentyl-4,5-diphenylimidazol, 2-Cyclohexyl-4,5-diphenylimidazol, 2,4,5-Triphenylimidazol, 2-(p-Nitrophenyl)-4,5-diphenylimidazol oder 2-Phenyl-4,5-bis-(p-nitrophenyl)-imidazol.

Durch Umsetzung der Phenylimidazole II mit Salpetersäure-/Schwefelsäuregemischen erhält man die entsprechenden p-Nitrophenyl-imidazole I. Neu und besonders vorteilhaft ist die Durchführung der Reaktion in Gegenwart von Harnstoff.

Harnstoff kann dabei in fester Form oder in Form von wäßrigen Lösungen, die z.B. 10 bis 60 Gew.% Harnstoff enthalten, eingesetzt werden. Im allgemeinen enthalten die Reaktionslösungen 0,02 bis 3 Mol, bevorzugt 0,1 bis 1 Mol Harnstoff, bezogen auf die Phenylimidazole II.

Die Reaktion kann diskontinuierlich oder kontinuierlich in den dafür geeigneten Apparaturen durchgeführt werden. Bei diskontinuierlicher Durchführung wird z.B. das Ausgangsprodukt zusammen mit dem Harnstoff in Schwefelsäure gelöst und anschließend zu dieser Lösung Salpetersäure zugefahren.

Salpetersäure kann z.B. als 35 bis 100%ige Salpetersäure eingesetzt werden. Vorteilhaft verwendet man technische, konzentrierte, 63 bis 68%ige Salpetersäure.

Salpetersäure wird in mindestens stöchiometrischen Mengen, beispielsweise 1 bis 5 Äquivalente, bevorzugt 1 bis 1,5 Äquivalente, bezogen auf die Anzahl der zu nitrierenden Phenylreste, eingesetzt.

Schwefelsäure kann z.B. als 90 bis 100%ige Schwefelsäure oder als Oleum mit Gehalten bis zu 65% an Schwefeltrioxid eingesetzt werden. Bevorzugt verwendet man technische, konzentrierte 96 bis 98%ige Schwefelsäure.

Die Menge an Schwefelsäure ist nicht besonders kritisch. Üblicherweise wird man die Menge so wählen, daß ein gut rührbares Reaktionsgemisch entsteht, beispielsweise die ein- bis vierfache Gewichtsmenge, bezogen auf die zu nitrierende Phenylimidazolverbindung II.

Die Reaktion wird bei Temperaturen von 20 bis 220°C , bevorzugt 40 bis 140°C, besonders bevorzugt 50 bis 120°C durchgeführt.

Durch das erfindungsgemäße Verfahren werden die p-Nitrophenyl-imidazole I durchwegs in sehr guten Ausbeuten, häufig mit 90% oder mehr, erhalten.

Die Isolierung der p-Nitrophenyl-imidazole I erfolgt in an sich bekannter Weise, z.B. durch Verdünnen der Reaktionslösungen mit Wasser, Neutralisieren mit basischen Verbindungen und Abfiltrieren. Aufgrund des selektiven Verlaufs der Nitrierungsreaktion fallen die Produkte in hoher Reinheit an, weshalb sich eine weitere Aufarbeitung, z.B. durch Umkristallisieren, zumeist erübrigt.

Die erhaltenen p-Nitrophenyl-imidazole der Formel I sind wertvolle Zwischenprodukte, z.B. für die Herstellung von Kunststoffen.

Die folgenden Beispiele sollen die Erfindung näher erläutern, ohne sie einzuschränken.

### Beispiel 1

### Herstellung von 4,5-Bis-(4-nitrophenyl)-imidazol

110 g (0,5 Mol) 4,5-Diphenylimidazol und 15 g (0,25 Mol) Harnstoff wurden in 250 g konzentrierte Schwefelsäure (98%) eingetragen. Anschließend wurden 110 g konzentrierte Salpetersäure (63%) bei 70 bis 80°C zugetropft und 0,5 Stunden bei 70°C nachgerührt. Die erkaltete Reaktionsmischung wurde in 1 l Eiswasser eingegossen und mit konzentrierter Ammoniaklösung (25%) auf pH 10 gebracht. Das ausgefallene Produkt wurde abgesaugt und mit heißem Wasser gewaschen. Man erhielt 148 g (95,5%) 4,5-Bis-(4-nitrophenyl)-imidazol, Smp. 294 bis 296°C.

### Beispiel 2

### Herstellung von 2-Methyl-4,5-bis-(4-nitrophenyl)-imidazol

117 g (0,5 Mol) 2-Methyl-4,5-diphenylimidazol und 15 g (0,25 Mol) Harnstoff wurden in 270 g konzentrierte Schwefelsäure (98%) eingetragen. Anschließend wurden 110 g konzentrierte Salpetersäure (63%) bei 80 bis 90°C zugetropft und 2 Stunden bei 80°C nachgerührt. Die erkaltete Reaktionsmischung wurde in 1 l Eiswasser eingegossen und mit konzentrierter Ammoniaklösung (25%) auf pH 8 gebracht. Das ausgefallene Produkt wurde abgesaugt und mit heißem Wasser gewaschen. Man erhielt 150 g (92,6%) 2-Methyl-4,5-bis-(4-nitrophenyl)-imidazol, Smp. 230 bis 232°C.

### Beispiel 3

### Herstellung von 2,4,5-Tris-(4-nitrophenyl)-imidazol

148 g (0,5 Mol) 2,4,5-Triphenylimidazol und 15 g (0,25 Mol) Harnstoff wurden in 440 g konzentrierte Schwefelsäure (98%) eingetragen. Anschließend wurden 220 g konzentrierte Salpetersäure (63%) bei 60 bis 70°C zugetropft und 0,5 Stunden bei 60°C nachgerührt. Die erkaltete Reaktionsmischung wurde in 1,5 l Eiswasser eingegossen, das ausgefallene Produkt abgesaugt und mit heißem Wasser gewaschen. Man erhielt 197 g (91,4%) 2,4,5-Tris-(4-nitrophenyl)-imidazol, Smp. 320 bis 322°C.

### Beispiel 4

### Herstellung von 4(5)-(4-Nitrophenyl)-imidazol

72 g (0,5 Mol) 4(5)-Phenylimidazol und 15 g (0,25 Mol) Harnstoff wurden in 150 g konzentrierte Schwefelsäure (98%) eingetragen. Anschließend wurden 60 g konzentrierte Salpetersäure (63%) bei 60 bis 70°C zugetropft und 1 Stunde bei 60°C nachgerührt. Die erkaltete Reaktionsmischung wurde in 1 l Eiswasser eingegossen und mit konzentrierter Ammoniaklösung auf pH 7 gebracht. Das ausgefallene Produkt wurde abgesaugt und mit heißem Wasser gewaschen. Man erhielt 83 g (87,8%) 4(5)-(4-Nitrophenyl)-imidazol, Smp. 222 bis 224°C.

## Patentansprüche

1. Verfahren zur Herstellung von p-Nitrophenyl-imidazolen der allgemeinen Formel I 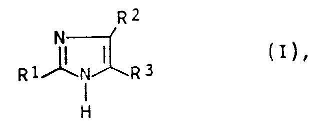 in der R¹ bis R³ unabhängig voneinander Wasserstoff, Alkyl, Cycloalkyl oder p-Nitrophenyl bedeuten, mit der Maßgabe, daß mindestens einer der Reste R¹ bis R³ für p-Nitrophenyl steht, durch Umsetzung von Phenylimidazolen der allgemeinen Formel II 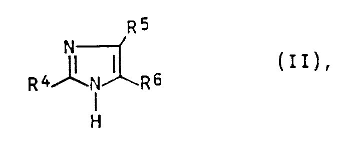 in der R⁴ bis R⁶ unabhängig voneinander Wasserstoff, Alkyl, Cycloalkyl, Phenyl oder p-Nitrophenyl bedeuten, mit der Maßgabe, daß mindestens einer der Reste R⁴ bis R⁶ für Phenyl steht, mit Gemischen von Schwefelsäure und Salpetersäure, dadurch gekennzeichnet, daß man die Reaktion in Gegenwart von 0,02 bis 3 Mol Harnstoff pro Mol Phenylimidazol durchführt.

## Claims

1. A process for the preparation of p-nitrophenyl-imidazoles of the general formula 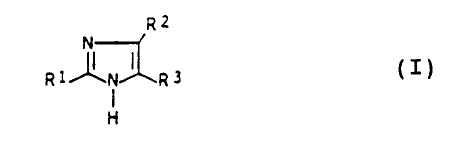 where R¹, R² and R³ independently of one another are hydrogen, alkyl, cycloalkyl or p-nitrophenyl, with the proviso that one or more of the radicals R¹, R² and R³ is p-nitrophenyl, by reacting a phenylimidazole of the general formula II 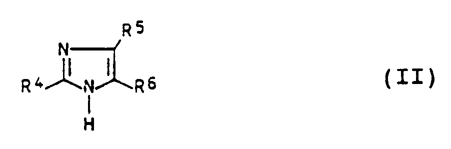 where R⁴, R⁵ and R⁶ independently of one another are hydrogen, alkyl, cycloalkyl, phenyl or p-nitrophenyl, with the proviso that one or more of the radicals R⁴, R⁵ and R⁶ is phenyl, with mixtures of sulfuric acid and nitric acid, which comprises carrying out the reaction in the presence of from 0.02 to 3 mol of urea per mole of phenylimidazole.

## Revendications

1. Procédé de préparation de p-nitrophénylimidazoles de la formule générale I 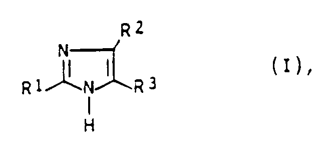 dans laquelle R¹ à R³ représentent, chacun indépendamment les uns des autres, un atome d'hydrogène, un radical alkyle, cycloalkyle ou p-nitrophényle, avec la condition qu'au moins l'un des substituants R¹ à R³ représente un radical p-nitrophényle, par la réaction de phénylimidazoles de la formule générale II 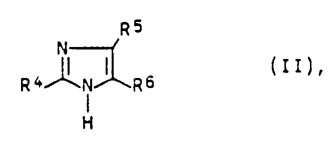 dans laquelle R⁴ à R⁶ représentent, chacun indépendamment les uns des autres, un atome d'hydrogène, un radical alkyle, cycloalkyle, phényle ou p-nitrophényle, avec la condition qu'au moins l'un des symboles R⁴ et R⁶ représente un radical phényle, avec des mélanges d'acide sulfurique et d'acide nitrique, caractérisé en ce que l'on entreprend la réaction en présence de 0,02 à 3 moles d'urée par mole de phénylimidazole.
